# EUROPEAN PATENT APPLICATION

(11) **EP 1 695 650 A1**
(43) Date of publication of application: **30.08.2006**
(21) Application number: 05356170.0
(22) Date of filing: 26.09.2005
(51) Int. Cl.: A47L 9/02, A61L 2/10, B08B 7/00

(54) **Suction nozzle having UV sterilizing lamp for vacuum cleaner and a method for manufacturing the same**

(30) Priority: 23.02.2005 KR 2005015068
(71) Applicant: Samsung Gwangju Electronics Co., Ltd., Gwangju-city (KR)
(72) Inventor: Lee, Hak-bong, Damyang-gun JeollaNamdo (KR); Oh, Jang-keun, Seo-gu Gwangju-city (KR); Seo, Ji-won, Gwangsan-gu Gwangju-city (KR)
(74) Representative: Myon, Gérard Jean-Pierre

(57) **Abstract**

A suction brush (20) and a method for manufacturing the same are provided. The suction brush includes an opening formed toward a surface being cleaned, the UV sterilizer lamp (40) is mounted at an inner space (23) of the opening to project a UV ray onto the surface, and a Teflon® window member (50) covering the inner space. The method includes mounting the UV sterilizer lamp at an inner space of an opening formed toward a surface being cleaned, connecting a Teflon® window member by fitting a plurality of connection projections (22a) formed around the opening into a plurality of connection holes formed at the Teflon® window member to correspond to the connection projections, determining a connection position for the Teflon® window member so that the inner space is ventilative, and fixing the Teflon® window member by compressing ends of the plurality of connection projections as penetrating the connection holes by heat and pressure.

## Description

### BACKGROUND OF THE INVENTION

### 1. Field of the invention

The present invention relates to a vacuum cleaner. More particularly, the present invention relates to a vacuum cleaner suction brush having an ultraviolet (UV) sterilizer for disinfecting a surface being cleaned, and a method for manufacturing the same.

### 2. Description of the Related Art

Generally, a vacuum cleaner collects dust on a surface being cleaned by drawing in ambient air, using a negative pressure generated by a vacuum suction means mounted in a cleaner body. With the booming of well-being and healthy life-styles, recently, a vacuum cleaner having an ultraviolet (UV) sterilizer provided to the cleaner body has been introduced in order to remove germs which may exist in the dust collected in a dust receptacle as well as merely collecting the dust. As a related art, Korean Utility Model Laid-open No. 2001-0001345 discloses a suction brush for a vacuum cleaner, in which a UV sterilizer lamp is mounted in a lower casing having a dust suction hole for drawing in dust-laden air from a surface being cleaned.

However, when any obstacle exists on a path of a UV ray projected from the UV sterilizer lamp, the UV ray is not able to pass through the obstacle. In order to minimize loss of the UV ray generated in the UV sterilizer lamp and projected onto a surface being cleaned, an opening is kept open or a grill for preventing damage of the UV sterilizer lamp by rough dust may be equipped with the opening.

When the UV sterilizer lamp is exposed as the above, however, the dust being collected may contaminate or damage the UV sterilizer lamp as time elapses. Especially, when a surface of the UV sterilizer lamp is polluted, the UV ray, being blocked by the dust attached on the surface of the UV sterilizer lamp, cannot be entirety transmitted to the surface being cleaned. Accordingly, a quartz glass having superior transmittance has been introduced to seal the opening and thereby prevent contamination of the UV sterilizer lamp.

While the quartz glass sealing the opening is able to protect the UV sterilizer lamp from the drawn-in dust, heat generated by the UV sterilizer lamp cannot be discharged to the outside. Accordingly, the UV sterilizer lamp may be overheated and therefore be broken since solidity of the quartz glass is not high enough to endure the heat.

### SUMMARY OF THE INVENTION

An aspect of the present invention is to solve at least the above problems and/or disadvantages and to provide at least the advantages described below. Accordingly, an aspect of the present invention is to provide a suction brush equipped with an ultraviolet (UV) sterilizer lamp, which is capable of interrupting impurities drawn in from a surface being cleaned, and a method for manufacturing the same.

Another aspect of the present invention is to provide a suction brush having an improved mounting structure to solve overheating of the UV sterilizer lamp, and a method for manufacturing the same.

In order to achieve the above-described and other aspects of the present invention, there is provided a suction brush for a vacuum cleaner. The suction brush has a UV sterilizer lamp, comprising an opening formed toward a surface being cleaned, the UV sterilizer lamp mounted at an inner space of the opening to project a UV ray onto the surface being cleaned, and a Teflon® window member covering the inner space of the opening.

The Teflon® window member is connected at a predetermined distance from the opening for ventilation of the inner space.

The inner space has therein a specular surface for focusing the UV ray generated by the UV sterilizer lamp upon the surface being cleaned.

Here, the specular surface is formed to widen toward the surface being cleaned. The specular surface may be implemented by attaching an aluminum tape.

A plurality of connection projections are formed around the opening while a plurality of connection holes are formed at the Teflon® window member to correspond to the connection projections. The plurality of connection projections may be formed as cylindrical columns arranged in a row, or may be formed as polygonal columns arranged in a plurality of rows so that the connection projections in one row face those in another row in an alternate manner.

A method for manufacturing the suction brush having the UV sterilizer lamp, according to an embodiment of the present invention, comprises the steps of mounting the UV sterilizer lamp at an inner space of an opening formed toward a surface being cleaned, connecting a Teflon® window member by fitting a plurality of connection projections formed around the opening into a plurality of connection holes formed at the Teflon® window member to correspond to the connection projections, determining a connection position for the Teflon® window member so that the inner space of the opening is ventilative, and fixing the Teflon® window member by compressing ends of the plurality of connection projections as penetrating the connection holes by heat and pressure.

### BRIEF DESCRIPTION OF THE DRAWING FIGURES

The above aspect and other features of the present invention will become more apparent by describing in detail exemplary embodiments thereof with reference to the attached drawing figures, wherein;

FIG. 1 is a perspective view showing an exemplary upright-type vacuum cleaner;

FIG. 2 is a bottom view of a suction brush comprising an ultraviolet (UV) sterilizer lamp, according to the present invention;

FIG. 3 schematically shows the inner structure of an opening being equipped with the UV sterilizer lamp;

FIG. 4 illustrates the connection structure of a Teflon® window member, according to a first embodiment of the present invention;

FIGS. 5 and 6 schematically show the inner structure and illustrate the connection structure of a Teflon® window member, according to a second embodiment of the present invention;

FIG. 7 is a flowchart explaining a method of constructing a suction brush according to the present invention; and

FIGS. 8, 9 and 10 are enlarged view showing main parts of FIG. 4, illustrating processes of connecting the Teflon® window member.

### DETAILED DESCRIPTION OF THE EXEMPLARY EMBODIMENTS

Hereinafter, certain embodiments of the present invention will be described in detail with reference to the accompanying drawing figures.

In the following description, same drawing reference numerals are used for the same elements even in different drawings. The matters defined in the description such as a detailed construction and elements are nothing but the ones provided to assist in a comprehensive understanding of the invention. Thus, it is apparent that the present invention can be carried out without those defined matters. Also, well-known functions or constructions are not described in detail since they would obscure the invention in unnecessary detail.

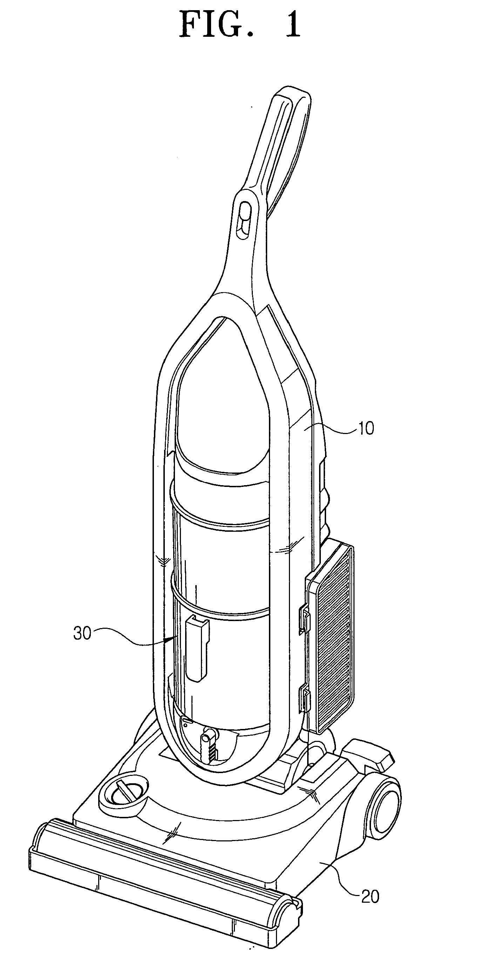

As shown in FIG. 1, an upright-type vacuum cleaner includes a cleaner body 10 and a suction brush 20 connected to the cleaner body 10 to draw in dust-laden air by moving along a surface being cleaned. In addition, a dust collecting apparatus 30 is removably mounted in the cleaner body 10 to collect therein dust drawn in through the suction brush 20 and separated from the dust-laden air.

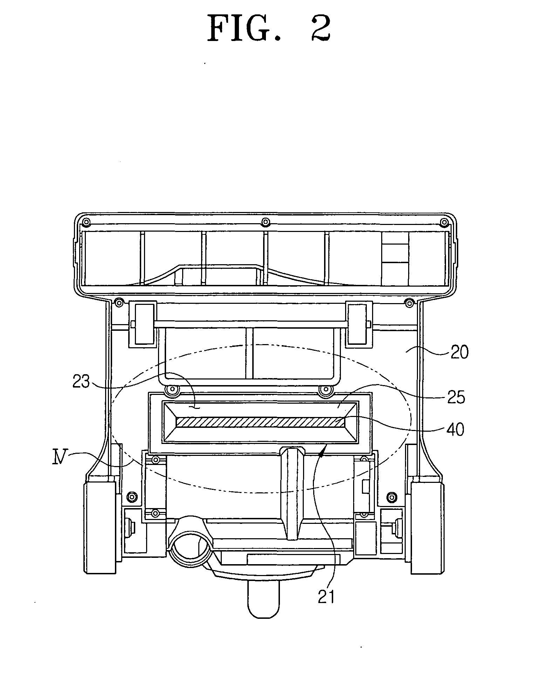

The suction brush 20 comprises, as shown in FIG. 2, an opening 21 formed toward the surface being cleaned and an ultraviolet (UV) sterilizer lamp 40 formed at an inner space 23 of the opening 21 to generate a UV ray.

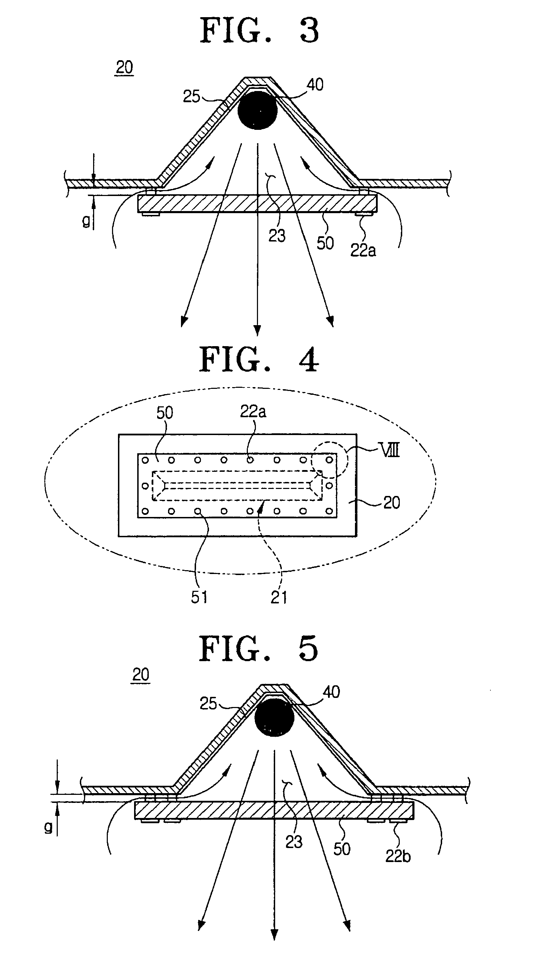

According to a first embodiment of the present invention, as shown in FIGS. 3 and 4, a specular surface 25 is formed in the inner space 23 to focus the generated UV ray upon the surface being cleaned. The specular surface 25 is formed to widen toward the surface being cleaned so as to converge the UV ray generated in the UV sterilizer lamp 40 as much as possible and then evenly distribute the converged UV ray onto the surface being cleaned. The specular surface 25 is made of a material having superior reflexibility. Therefore, the specular surface 25 may be implemented by attaching an aluminum tape on a wall of the inner space 23.

A transparent Teflon® window member 50 is connected to the opening 21 at a predetermined distance 'g' from the opening 21 in order to prevent the UV sterilizer lamp 40 mounted at the inner space 23 from being contaminated by the drawn-in dust. Since the Teflon® has superior transmittance, the Teflon® window member 50 does not have to be transparent. However, for a user to easily observe the state of the UV sterilizer lamp 40 with unaided eye, it is preferable to form the Teflon® window member 50 of a transparent material. As shown in FIG. 4, the Teflon® window member 50 is fixed by conical connection projections 22a arranged in a row.

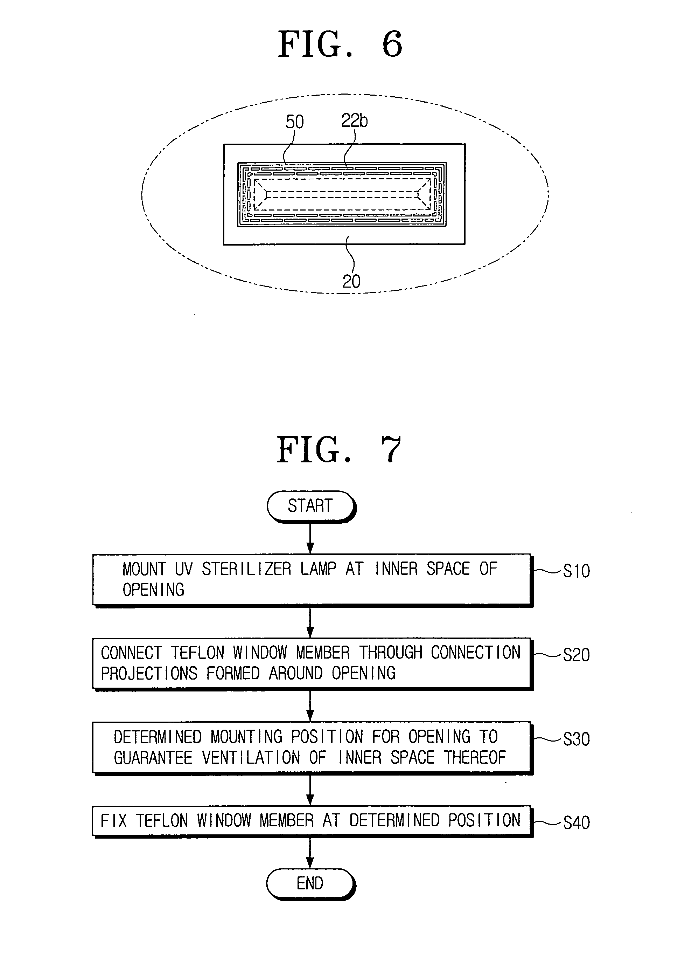

FIGS. 5 and 6 illustrate a second embodiment of the present invention, which has the similar construction with the first embodiment described above. More specifically, the UV sterilizer lamp 40 is mounted at the inner space 23 and the Teflon® window member 50, which may be transparent, is mounted to the opening 21 at a predetermined distance from the opening 21. The distinctive feature of the second embodiment is configuration of the connection projections formed around the opening 21. As shown in the drawings, the connection projections 22b are formed as polygonal pillars arranged in a plurality of rows. The connection projections 22b in one row face those in another row in an alternate manner. By this structure, compared to the first embodiment, the opening 21 and the Teflon® window member 50 can be more firmly connected with each other.

The connection projections 22a and 22b of the first and the second embodiments are extendedly formed from the suction brush 20. Therefore, the suction brush 20 may be integrally formed with the connection projections 22a or 22b by injection molding.

Hereinbelow, a method of constructing the suction brush having a UV sterilizer lamp will be described with reference to FIGS. 7, 8 and 10. Since the method of connecting the Teflon® window member 50 with the suction brush 20 is applied in the same manner to both the first and the second embodiments, the connection method will be described with respect to only the first embodiment.

As shown in FIGS. 3 and 4, the opening 21, being opened toward the surface being cleaned, is formed on a bottom of the suction brush 20. The UV sterilizer lamp 40 is mounted at the inner space 23 of the opening 21 (S10).

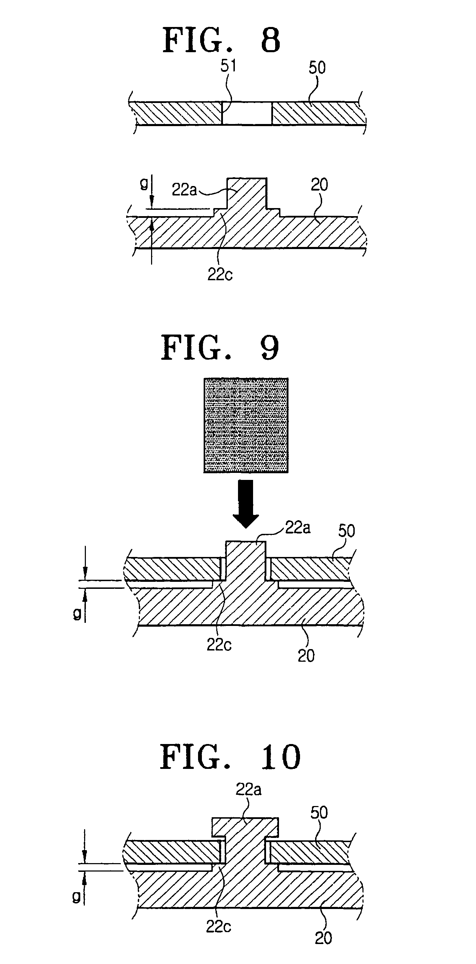

The plurality of connection projections 22a are formed around the opening 21, as shown in FIG. 8. On the other hand, a plurality of connection holes 51 are formed corresponding to the connection projections 22a at the Teflon® window member 50 covering the opening 21. The connection projections 22a and the connection holes 51 are engaged with each other so that the Teflon® window member 50 covers the opening 21 (S20)

As shown in FIG. 9, the Teflon® window member 50 is disposed at a predetermined distance 'g' from the opening 21 so that the heat generated in the inner space 23 can be discharged to the outside. Preferably, the distance 'g' is approximately 1mm (S30). For example, each of the plurality of connection projections 22 can include a shoulder 22c for supporting the Teflon® window member 50 at the predetermined distance 'g' from the opening 21 as shown in FIGS 8 through 10.

When the connection position of the Teflon® window member 50 is determined, each end of the plurality of connection projections 22a as penetrating the plurality of connection holes 51 is compressed by heat and pressure, thereby fixing the Teflon® window member 50, as shown in FIG. 10 (S40). Because the Teflon® material seldom adheres to other substances by adhesive due to its low affinity, the Teflon® window member 50 is fixed in the above-described manner.

Although not explained in detail, in the second embodiment as well, ends of the connection projections 22b as penetrating the connection holes 51 are compressed by heat and pressure to thereby fix the Teflon® window member 50 at the determined position.

Using the transparent Teflon® window member 50 as described above, robustness against impact can be improved compared to the conventional art using a quartz glass for the window member. In addition, since the Teflon® window member 50 protecting the UV sterilizer lamp 40 from dust or obstacles is connected to the opening 21 mounting therein the UV sterilizer lamp 40 in a ventilative manner, the inner space 23 including the UV sterilizer lamp 40 is not completely sealed. Therefore, the heat generated by the UV sterilizer lamp 40 can be discharged to the outside, thereby preventing malfunction of the UV sterilizer lamp 40 due to the high temperature.

Furthermore, by forming the window member of the transparent Teflon® resin having superior transmittance and solidity, reliability of the window member can be improved compared to the conventional quartz glass window member.

While the invention has been shown and described with reference to certain embodiments thereof, it will be understood by those skilled in the art that various changes in form and details may be made therein without departing from the spirit and scope of the invention as defined by the appended claims.

## Claims

1. A suction brush (20) for a vacuum cleaner, which is provided with an ultraviolet (UV) sterilizer lamp (40), the suction brush comprising:
an opening (21) formed toward a surface being cleaned;
the UV sterilizer lamp mounted at an inner space (23) of the opening to project a UV ray onto the surface being cleaned; and
a Teflon® window member (50) covering the inner space of the opening.

2. The suction brush of claim 1, wherein the Teflon® window member (50) is connected at a predetermined distance (g) from the opening (21) for ventilation of the inner space (23).

3. The suction brush of claim 1, wherein the inner space (23) has therein a specular surface (25) for focusing the UV ray generated by the UV sterilizer lamp (40) upon the surface being cleaned.

4. The suction brush of claim 3, wherein the specular surface (25) is formed to widen toward the surface being cleaned.

5. The suction brush of claim 4, wherein the specular surface (25) comprises an aluminum tape.

6. The suction brush of claim 3, wherein the specular surface (25) comprises an aluminum tape.

7. The suction brush of claim 1, further comprises a plurality of connection projections (22a, 22b) are formed around the opening (21) while a plurality of connection holes (51) are formed at the Teflon® window member (50) to correspond to the plurality of connection projections.

8. The suction brush of claim 7, wherein the plurality of connection projections (22a) are formed as cylindrical columns arranged in a row.

9. The suction brush of claim 7, wherein the plurality of connection projections (22b) are formed as polygonal columns arranged in a plurality of rows so that the plurality of connection projections in one row face those in another row in an alternate manner.

10. A method for manufacturing a suction brush (20) having a UV sterilizer lamp (40), the method comprising the steps of:
mounting (S10) the UV sterilizer lamp (40) at an inner space (23) of an opening (21) formed toward a surface being cleaned;
connecting (S20) a Teflon® window member (50) by fitting a plurality of connection projections (22a, 22b) formed around the opening (21) into a plurality of connection holes (51) formed at the Teflon® window member (50) to correspond to the connection projections;
determining (S30) a connection position for the Teflon® window member (50) so that the inner space (23) of the opening (21) is ventilative; and
fixing (S40) the Teflon® window member (50) by compressing ends of the plurality of connection projections (22a, 22b) as penetrating the connection holes (51) by heat and pressure.

11. A suction brush (20) for a vacuum cleaner comprising:
an opening (21) facing toward a surface to be cleaned;
a plurality of connection projections (22a, 22b) around the opening extending toward the surface;
an ultraviolet (UV) sterilizer lamp (40) mounted in the opening to project a UV ray towards the surface;
a window member (50) having a plurality of connection holes (51), each of the plurality of connection holes receiving a respective one of the plurality connection projections; and
a compressed end defined at each of the plurality connection projections so that the window member is secured over the opening.

12. The suction brush of claim 11, wherein the plurality of connection projections (22a) are formed as cylindrical columns arranged in a row.

13. The suction brush of claim 11, wherein the plurality of connection projections (22b) are formed as polygonal columns arranged in a plurality of rows so that the connection projections in one row face those in another row in an alternate manner.

14. The suction brush of claim 11, wherein the window member (50) comprises Teflon® .

15. The suction brush of claim 14, wherein the window member (50) is or is not transparent.

16. The suction brush of claim 11, wherein the plurality of connection projections (22a) each include a shoulder (22c) for connecting the window member at a predetermined distance (g) from the opening.

17. The suction brush of claim 11, wherein the predetermined distance (g) comprises approximately 1mm.
